# EUROPEAN PATENT SPECIFICATION

(11) **EP 3 835 098 B1**
(45) Date of publication and mention of the grant of the patent: **04.05.2022**
(21) Application number: 20212792.4
(22) Date of filing: 09.12.2020
(51) Int. Cl.: B60H 1/00, B60H 3/06, A61L 9/20, B01D 53/88

(54) **DEVICE FOR SANITISING THE AIR INSIDE A VEHICLE**
EINRICHTUNG ZUR REINIGUNG DER LUFT INNERHALB EINES FAHRZEUGS
APPAREIL DE NETTOYAGE DE L'AIR À L'INTÉRIEUR D'UN VÉHICULE

(30) Priority: 12.12.2019 IT 201900023847
(43) Date of publication of application: 16.06.2021
(73) Proprietor: Ellamp S.p.A., 21020 Bodio Lomnago (VA) (IT)
(72) Inventor: OTTINO, Massimo, 21100 Daverio (Varese) (IT); TREPICCIONE, Nicola, 21028 Travedona Monate (Varese) (IT)
(74) Representative: Petruzziello, Aldo

(56) References cited:
- JP-A- 2000 060 947
- JP-A- 2000 060 948
- US-A1- 2018 056 758
- US-B1- 6 468 491

## Description

The present invention relates to a device for sanitising by photocatalysis the air inside a vehicle, in particular a means of public transport. More specifically, the present invention relates to a device to be applied to the air conditioning aspiration intakes and delivery outlets to reduce the concentration of pathogens and in general to improve air quality in the internal spaces of a bus or the like by means of photocatalysis.

### Background of the invention

Road and rail public transport is generally equipped with features that significantly increase the level of comfort of the passengers and provide numerous functional solutions and details that improve and facilitate the travel experience for the occupants. Vehicle interiors, for this purpose, provide configurations generally enriched with content that improves the level of passenger comfort at various levels and in various ways. The use of more sophisticated upholstery and fabrics that improve acoustic and tactile comfort, the use of construction systems such as to guarantee larger and easier to reach loading areas for hand luggage, higher performance passenger services in terms of reading light and entertainment and attention to better quality of the air diffused in the passenger compartment, for example, are all services designed to improve the psychological and physical wellbeing of passengers.

The psychological and physical wellbeing of passengers is influenced not only by the perceived sensation of comfort, but also by the healthiness of the space and of the air in which passengers are immersed during the journey.

In the means of transport, however, the channels used for the diffusion of air conditioning often do not undergo maintenance (net of the replacement of dust filters to be performed periodically) and can easily become prey to microorganisms such as bacteria, mould and fungi. These viruses, thanks to the entrainment of air, significantly increase the potential contagion among people present in the same environment, with the risk of causing the spread of diseases and allergies.

In this regard, the organisations in charge of public health control have supplied precise indications on the consequences deriving from poor air quality in a closed environment, whose low level of healthiness is determined both by the high level of external pollution and the numerous internal polluting sources. It is therefore necessary to "reclaim" the air inside the passenger compartments of means of transport.

There are currently various air purification technologies, among which the most used are thermodynamic sterilisation, ultraviolet irradiation, the use of active carbon filters, electronic polarisation, ionisation and ozonisation. All these technologies have some weak points: limited efficiency in the abatement of pathogens, the need to carry out these operations with the vehicle unloaded, reduction in efficacy over time due to the deterioration of the active component, etc.

One of the most widely used devices today, for example, is represented by portable ozonisers, whose main function is the production of ozone, i.e. a molecule composed of three oxygen atoms. It is a strong oxidiser, which is therefore able to oxidise and consequently destroy bacteria, viruses and other germs. However, its oxidising effect is not limited to the external environment: if it is present in too high a concentration, in fact, it can also be harmful to the human organism.

This means that the sanitisation operation requires a certain level of safety and must be carried out with the vehicle empty, since a certain amount of time is needed so that the ozone concentration can fall to levels such as not to compromise the health of the occupants. In addition, portable ozonisers do not form an integral part of the vehicle's system and their use could therefore be occasional, invasive and not guarantee continuity in the healthiness of the air.

In addition to ozonisation systems, photocatalysis devices have recently been developed, used with the aim of sanitising the air. Photocatalysis is a natural phenomenon in which a nanomaterial substance, known as photocatalyst, through the action of light - natural or artificial - gives rise to a chemical reaction that involves the production of radicals, i.e. oxidising substances capable of attacking the defences of pathogenic elements and breaking them down at a molecular level, having as the end result of this process only non-harmful products such as water vapour, carbon dioxide, calcium carbonate, etc.

The nanomaterial most commonly studied and used for photocatalysis is currently titanium dioxide. Despite its excellent sanitising capacity, however, this substance has the disadvantage that it is only activated when illuminated with ultraviolet radiation, which is not available in sufficient quantities in closed internal environments. In order to produce its effects, titanium dioxide therefore needs to be activated through the use of specific artificial sources which are dangerous for the occupants - if directly exposed to them - and not very economical from a construction point of view.

Therefore, although capable of performing a very important action from the point of view of sanitisation, devices that exploit the effect of photocatalysis are still not widespread on board vehicles in that not very advantageous.

US 2018/0056758 describes a device for directing a flow of light and a flow of air towards the passenger compartment of a vehicle. The entire device is formed by a duct for distributing air in output from an air conditioning system, and the duct has an opening at its end wherein a series of deflectors are attached, which in one embodiment can be lamellae provided with a titanium dioxide (TiO₂) based photocatalytic coating. Inside the duct a light source is provided which, by means of appropriate functional modifications in the duct itself, allows the light flow to be directed outwards and therefore onto the lamellae.

Patent US 6468491 B1 describes a dashboard positioned at the mouth of air intakes or vents of an installation intended for heating, the delivery of air conditioning or ventilation of a vehicle, in particular a car. The dashboard has an upper wall provided with a series of orifices for the circulation of the air, which is covered by a layer of photocatalytic material, preferably TiO₂. The dashboard extends for its full width over a diffusion chamber of the installation, inside of which one or more UV (ultraviolet) lamps are provided. The action of the light emitted by the UV lamps on the photocatalytic layer allows a purifying effect to be obtained on the air.

The devices described above use titanium dioxide as a photocatalyst which, in order to be effective in terms of abatement of pollutants, requires a highly energetic source such as in fact UV rays and, more generally, requires artificial sources that are dangerous for the occupants, uneconomical and not very versatile from the point of view of construction, as well as not very durable over time.

In order to limit the harmful effect of UV rays coming out of the orifices of the device, special means are provided in US6468491B1 for blocking them when the lamps are in operation. In a particular embodiment of the device, in which the dashboard is presented as a series of lamellae delimiting the orifices, the lamellae must be carefully oriented so that the light coming from the UV lamp installed inside the diffusion chamber of the air circulation system only hits the lower face so that it does not spread between the orifices.

It has recently been found that nanomaterials containing tungsten trioxide (WO₃) are more effective than any other antibacterial agent and other photocatalytic materials, such as titanium dioxide, which react mainly to ultraviolet rays.

For example, JP 2000 060947A discloses an air purifier with a casing installed on a passenger-car rear tray as an on-vehicle equipment. The casing has in its top a window that allows the sunlight to pass through, and includes a photocatalyst carrier disposed under the window. Sunlight coming in through the rear window passes through the window and activates the photocatalyst, which in turn offers an air purifying effect. In some embodiments, tungsten trioxide (WO₃) is used as a photocatalyst material.

The use of WO₃ as a photocatalyst material is also known from JP 2000 060948A, which discloses an air purifier of a type wherein its casing is set on the floor of an automobile between the driver's and front passenger seats. The casing has an inlet port in its front, a vent port in its back, and an internal air duct leading from the inlet port to the vent port and it is also internally mounted with a dust-collecting filter, a pair of photocatalyst carriers, a light source and a blower arranged downstream in this order.

The particular nature and great convenience of this nanomaterial consists in the fact that the photocatalysis reaction is initiated in the visible spectrum. In fact, it has been found that materials containing tungsten trioxide applied on LED lights (which, being directional light, allows the highest illuminance emission to be obtained on the treated surface) remain always active when the light is on, and are able to eliminate almost all viruses, bacteria and other harmful substances present in the environment in less time than known sanitising devices. On the contrary, the reactivity of TiO₂ to visible light is not such as to be effective in terms of purification in non-doped compositions. To reach acceptable levels of sensitivity it is necessary to enrich the composition with noble elements such as platinum in high percentages.

The characteristics of tungsten trioxide eliminate in fact the disadvantages of the photocatalysts described above and make it possible to consider use on board both urban and tour vehicles of sanitising devices that exploit photocatalysis with decidedly high efficiencies combined with the use of internal light or economic and efficient artificial sources (e.g. LED lamps), as well as direct sunlight where possible.

It should also be noted that photocatalytic purification devices, known for example from US 2018/0056758 and from US6468491B1, provide as a support for the photocatalyst integral parts of the vehicle finishes that are unprotected and exposed to dust deposits which over time prevent the light source from being able to energise the photocatalyst and thus give rise to surface chemical reactions aimed at the formation of oxidising elements.

They are also created as an integral part of the first ventilation or air conditioning system, which makes them difficult to replace and completely inapplicable subsequently on vehicles that do not have them.

### Summary of the invention

The object of the present invention is therefore to provide a device for sanitising by photocatalysis the air inside a vehicle, such as a bus or similar means of transport, in order to reduce the bacterial load in the interior spaces of the vehicle.

Another object of the present invention is to provide a device for sanitising by sis the air inside a vehicle, that can be retrofitted and in a replaceable manner at the ends of the intake and delivery ducts of the air conditioning and/or recirculation of air without changing the layout of the vehicle.

A further object of the present invention is to provide a device for sanitising by photocatalysis the air inside a vehicle, capable of achieving greater results in terms of efficiency, safety and durability with respect to currently known systems based on the principle of photocatalysis.

A further object again is to provide a device for sanitising by photocatalysis the air inside a vehicle, with a modular and scalable configuration so that it can be applied to various vehicles with different layouts. Finally, one object of the present invention is to provide a device for sanitising by photocatalysis the air inside a vehicle, that is versatile, economical and easy to produce.

These and other objects are achieved by a device in accordance with the invention having the features listed in the appended independent claim 1. Advantageous embodiments of the invention are disclosed by the dependent claims.

### Brief description of the drawings

Further features of the invention will be made clearer by the detailed description that follows, referring to a purely illustrative, and therefore not limiting, embodiment illustrated in the accompanying drawings, in which:
Figure 1 is an axonometric view from above (i.e. from the air outlet side) of an air sanitising device according to the present invention;
Figure 1a is an enlarged view of the detail enclosed in circle C in Figure 1;
Figure 2a is an axonometric view of a lamellar module of the sanitising device according to the present invention;
Figure 2b is a plan view from above of the lamellar module of Figure 2a;
Figure 2c is a section view taken along line A-A of Figure 2b;
Figure 2d is an enlargement of the detail denoted by B in Figure 2b;
Figures 3a, 3b, 3c, 3d are, respectively, a side profile view, plan view, from above, from the left side of Figure 3a, and from below of the sanitising device according to the present invention;
Figure 4a is a section view taken along line A-A in Figure 3b;
Figure 4b is a section view taken along line B-B in Figure 3b;
Figure 4c is an enlargement of the detail denoted by D in Figure 4a;
Figures 5a and 5b are plan views from above showing two groups of lamellar grilles comprising, respectively, three and six lamellar modules; and
Figure 6 is a schematic representation showing the sanitising device according to the present invention applied to the circulation and delivery system of the air inside a vehicle.

### Detailed description of the invention

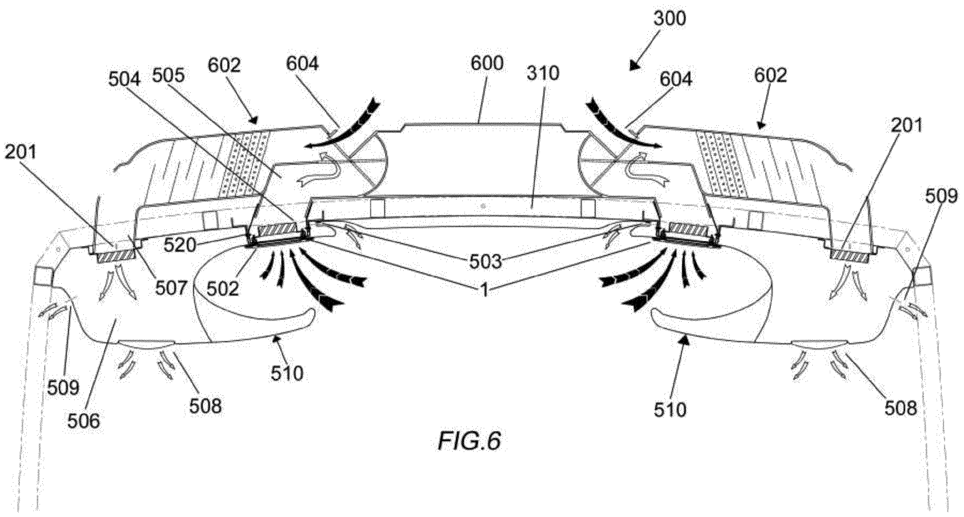

Referring to the accompanying drawings, a preferred embodiment of the sanitising device according to the invention and, referring in particular to Figure 6, an example of its application in an air circulation system in a vehicle will now be described in detail.

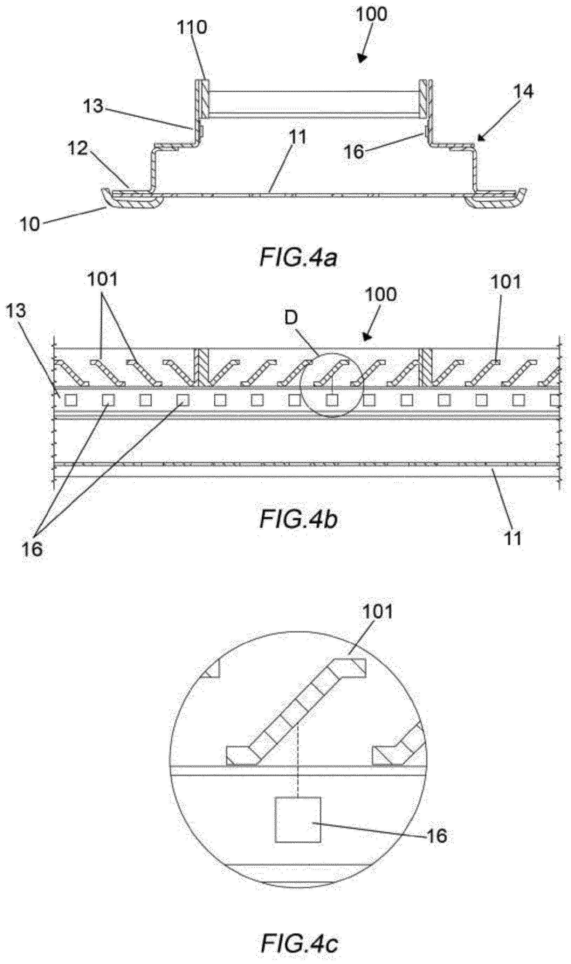

The device for sanitising by means of photocatalysis, denoted as a whole by reference numeral 1 or 201 (Fig. 6), substantially comprises a mechanical support structure 14 for a series of lamellar modules 100 containing a photocatalytic material and for a light source 16 (Figures 4a-c), specifically an LED light, suitable for illuminating and activating the photocatalytic material in order to obtain a sanitising effect of the air that traverses the grilles of the modules.

The support structure 14 is suitable to be installed in intakes and on the standard air conditioning delivery lines present on public transport vehicles, understanding intakes to be the openings designed to collect exhausted air to be sanitised from inside the vehicle and delivery lines the air conditioning lines downstream of an air conditioning unit.

The structures traditionally used, when installed in an air intake, generally have an aesthetically pleasing openable grille - formed by an elongated base body on which a perforated metal sheet is applied - equipped with a filter and a filter holder contained in a small frame fixed to the base. When they are installed in the delivery line, the structures are not visible and therefore do not have an aesthetic function. In these cases there is no base body.

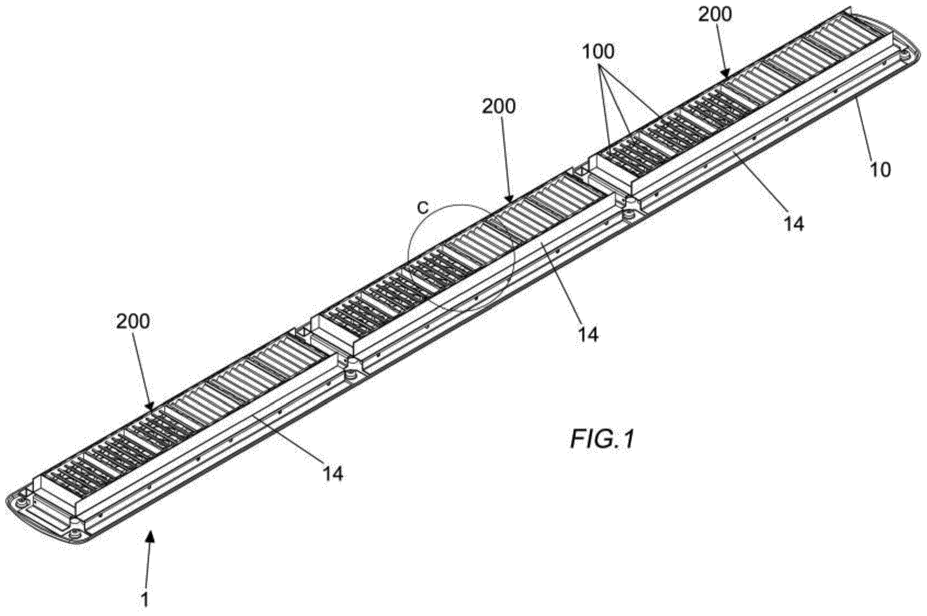

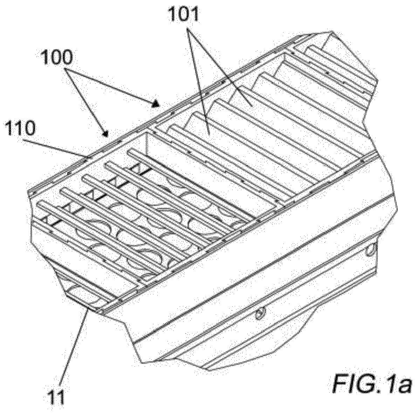

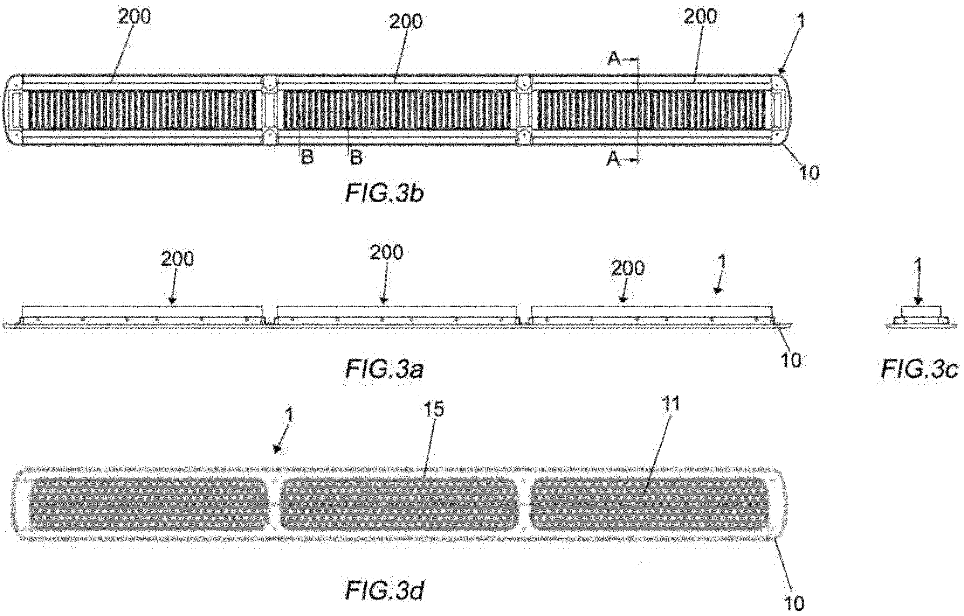

Fig. 1 shows a perspective representation of a sanitising device 1 seen from above, having the lamellar modules 100 in evidence, intended to be applied in air intakes. In the lower part of the device there is, in fact, in a conventional manner, a perforated grille 11, which can be seen in the detail shown in Fig. 1a and shown more clearly in the plan view of Fig. 3d.

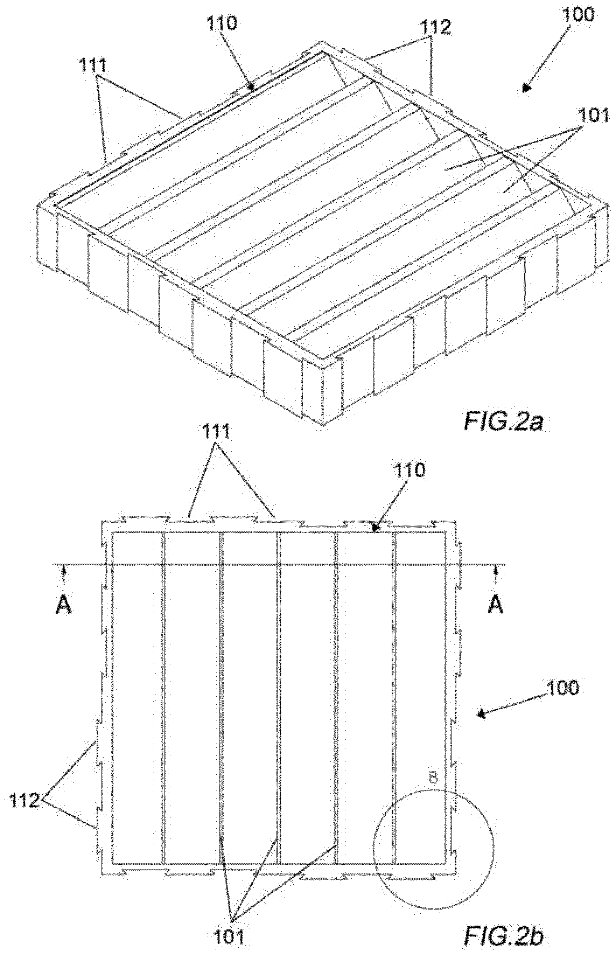

A lamellar module 100, shown isolated in the perspective view of Fig.2a, constitutes the element that performs the function of support for the photocatalytic material. The lamellar module 100 is formed substantially by an external perimeter frame 110 that encloses in its interior a series of lamellae 101 arranged parallel one to the other, so as to form a grille, as shown in the plan view from above of Fig. 2b.

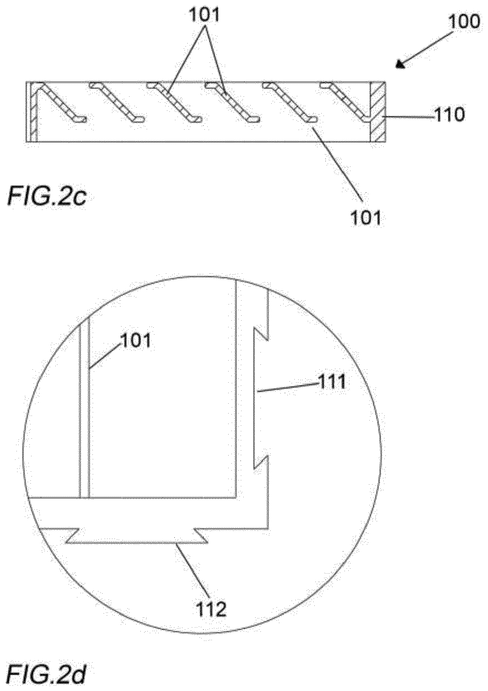

Fig. 2c, which is a section view taken along line A-A of Fig. 2b, also shows that the individual lamellae 101 are inclined at a certain angle with respect to the frame of the lamellar module 100, so as to conventionally define a face turned downwards and a face turned upwards for lamellae 101. The inclination shown in Fig. 2c is purely an example, and the lamellae could also be inclined in the opposite direction.

Referring to the orientation defined above of lamellae 101, the face turned downwards is the specific support for the photocatalytic material. This face of the lamellae 101 of module 100 is in fact treated, after the application of a primer, by deposition of a photocatalytic nanomaterial coating and represents the active surface wetted by air that gives rise to the chemical reactions necessary for the abatement of pathogens. That is to say, in use, this face is turned towards the entrance of the air to be sanitised.

The nanomaterial is represented by tungsten trioxide (WO₃), while the lamellar modules 100 preferably have a polymeric composition, in that very versatile and economical to manufacture from the production standpoint (by injection moulding).

However, the use of lamellar modules in other materials, such as metals or metal alloys, which have advantages in terms of treatment and deposition of the photocatalyst, is not excluded.

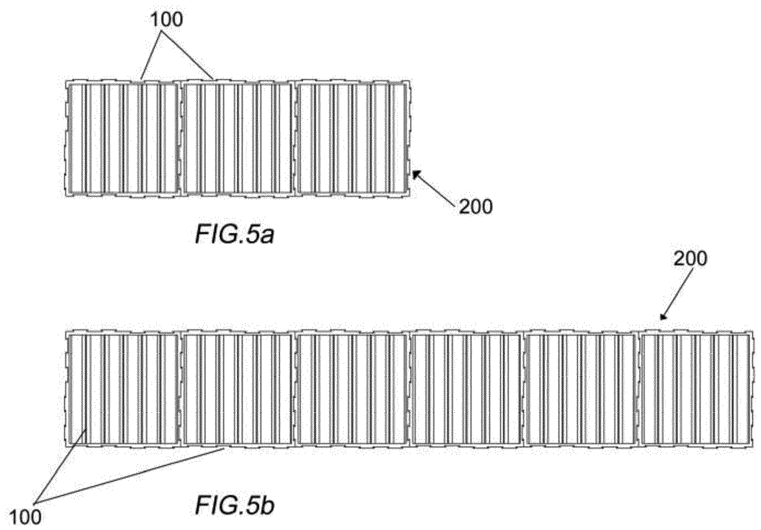

In a preferred embodiment, the lamellar module 100 has a square plan shape with a side of length equal to 100 mm and the lamellae 101 are arranged at a pitch of 16.67 mm. The lamellar modules 100 preferably have a frame 110 with knurled outer edge with recesses 111 and protrusions 112, as shown in Figs. 2a, 2b and in greater detail in Fig. 2d. This configuration favours the coupling of several lamellar modules 100 to obtain lamellar assemblies 200 like those shown in Fig. 5a, comprising three lamellar modules, or like the one shown in Fig. 5b, formed by six modules.

These representations are purely illustrative and the configuration of the lamellar modules 100 allows them to be combined in longitudinal and transversal sequence according to the size of the delivery and intake areas on the basis of the specific application, making the system highly scalable and adaptable on the basis of the various requirements.

In order to achieve the expected sanitising effect, the lamellar modules 100 equipped with a photocatalytic element must be coupled to a light source. The tungsten trioxide, which is the preferred nanomaterial for this purpose, is active in the visible spectrum and allows coupling with economical and efficient artificial sources such as LED light. To achieve the maximum hygienic effect, an LED source unit 16 must be placed at each lamella 101 of module 100 in order to ensure complete illuminance. For this reason, the axial pitch of lamellae 101 and the pitch of the sequence of light sources must preferably match.

The coupling between the light source and the lamellar modules 100 takes place via a support structure 14, which will now be illustrated in greater detail. When the sanitising device is installed at the air intakes, i.e. in points of the vehicle that remain visible, it also provides a support base 10 in order to guarantee the aesthetic effect and takes on the form illustrated in Figures 1 to 3d where it is denoted overall by reference numeral 1.

When, instead, the device is not visible, for example when it is applied on the air delivery line immediately downstream of an air conditioning unit, the sanitising device does not require the support base 10 and takes the shape of the device schematically shown in Figure 6, where it is denoted by numeral 201.

With particular reference to Figs. 3a to 4c, an embodiment of the sanitising device 1 is now described, comprising base 10 in itself conventionally known and suitable for insertion in the openings present on the vehicle chassis, for example in the roof area, necessary for air intake and recirculation.

Base 10 generally has an elongated configuration such as that shown from above - i.e. showing the lamellar modules 100 - in Fig. 3b and from below in Fig. 3d. Base 10 has one or more openings 15 for the passage of the air aspirated and its edge allows the application of a perforated grille 11. It is at the openings 15 that the lamellar assemblies 200 are in turn applied, in order to sanitise the air passing through grille 11.

The lamellar assemblies 200 can be formed by a different number of lamellar modules 100 according to the size of the openings 15 formed in the base 10. In the embodiment shown in Figs. 3a, 3b, 3d, solely by way of an example, lamellar assemblies comprising six modules 100 are used.

Fig. 4a, which is a section view taken along line A-A of Fig. 3b, illustrates the way in which the lamellar assemblies 200 comprising modules 100 are supported by support 14 and then attached to base 10.

The support structure 14 basically consists of a pair of elements, each of which is designed to be applied to the longitudinal edges of a lamellar assembly 200. For this purpose, each element of the support structure 14 has an upper section with at least one first face 13, suitable for housing the edge of unit 200 and the light source 16, and a lower section with at least one second face 12 inclined with respect to the first face 13, preferably perpendicular thereto, suitable for fixing to an air intake. In the case of sanitising device 201 without aesthetic function, the lower section can be applied directly to the air intake; in the case of sanitising device 1 to be installed on sight, instead, face 12 must first be coupled to base 10 carrying grille 11.

In the embodiment shown in the drawings, the support structure 14 consists of a pair of lower elements provided with a second face 12, in this case with S section, and a pair of upper elements provided with a first face 13, represented with an L section, made in separate parts. However, the configuration of support 14 may be different and be formed in a single piece, on the understanding that it must have at least one first face 13 to house the edge of the lamellar assembly 200 and one second face 12 to allow attachment to the air intakes.

The attachment and installation of the sanitising devices 1, 201 takes place in various traditional ways, for example by screwing of faces 12 to the air intakes.

The feature of the two upper elements of the support structure 14 is therefore that of having a sufficient surface area to support the edge of the lamellar assembly 200, in such a way that it is fixed on both sides, and to allow the attachment of the LED light source 16 below it, as can be seen more clearly in Fig. 4b and in the detail of Fig. 4c. A single LED 16 is placed at each lamella 101 of the lamellar modules 100 to make maximum use of the hygienic effect, which depends, among other things, on the light intensity and colour temperature of the source itself, as well as the inclination of the lamellae 101, which also have a light deflector effect.

The configuration described above thus allows attachment of the light source in the form of flexible LED strips. In the preferred embodiment, a 12-24 VDC flexible LED strip equipped with 60 LEDs per metre of white light (6000 - 6500 K) is used.

Naturally the configuration of the support structure 14 can be different from that shown in the drawings, in such a way that it can be adapted to different sizes of the lamellar assemblies 200 and of the support base 10 and can also be formed in one piece.

A sanitising device has been described above, comprising a series of compact lamellar assemblies, which are easy to build and suitable for insertion in a typical urban or tour vehicle architecture, such as the one shown in Fig. 6 purely by way of an example.

This drawing is a schematic representation of the upper part of a public transport vehicle 300 with an air conditioning unit 600 mounted on the roof 310 of the vehicle. Two conveyors 602 branch out from the air conditioning unit 600, each one intended to convey air into the vehicle at the passenger seats.

A pair of luggage racks 510 are placed inside the vehicle, attached to the roof 310 of the vehicle, above the seats. A standard luggage rack such as that shown in Fig. 6 has a loading area protruding towards the vehicle aisle and a covered area 506, window side, where a channel is developed for conveying air towards the passenger seats through an outlet 508. When one or more sanitising devices according to the invention are applied to the means of transport, the air leaving the outlet 508 is sanitised air.

Schematically, the exhausted air to be sanitised is taken from the inside of the vehicle and treated before it enters through air intakes 504 formed on the roof 310 in the air conditioning lines. These air intakes 504 are usually provided in a surface on which the luggage rack 510 is mounted, which therefore has an opening 502 at the aforementioned air intake 504 to allow suction. The opening 502, known in itself and suitable for allowing the insertion of conventional air recirculation grilles, allows the application of a sanitising device 1 equipped with a visible grille. In the configuration shown in Fig. 6, device 1 also partially performs the task of lighting the aisle of the transport means.

The air taken from inside the vehicle then first traverses the grilles of device 1, where it is purified of pathogens; subsequently it traverses the air intake 504 and enters an intake air channel 505 which develops in the conveyor 602. The air is directed towards channel 505 generally thanks to a channelling structure 520 that also allows the connection and continuity between the conveyor 602 and the opening 502 in the luggage rack 510. Sanitised air that is not channelled towards the air intake 504 can be immediately refed into the vehicle, for example by means of side slits 503 that introduce air towards the aisle.

The air collected in the aspiration channel 505 is then used by conditioning unit 600 to convey conditioned air to the passenger compartment via conveyor 602. In the diagram in Fig. 6, the conditioned air is fed into the vehicle through an outlet 507 formed in the roof 310, from which a channelling provided in the interior area 506 of the luggage rack 510 branches off, which carries the air directly onto the outlet 508 placed above the passenger seats.

During its functioning, the air conditioner may require external air, which enters the circuit via an air intake 604 provided on the conveyor unit 602. It often happens, therefore, that the air inside the conveyor is a mixture of sanitised air and untreated air.

For this purpose, an additional sanitising device 201 is placed at the outlet 507 and the zone of communication with the internal zone 510 of the luggage rack. Such a device 201, which has no aesthetic function and therefore only performs the purification task, can generally be provided at each air conditioning delivery point.

The configuration described above is solely an example and does not limit the application of the sanitising device to situations wherein the air conditioning unit is placed on the roof of the transport means or which have air channels inside the luggage racks. The air conditioners may, for example, be placed on the sides of the vehicle, in the window area, and the sanitising devices 1, 201 may be of a different number and applied in different positions from those described.

From what has just been illustrated, the advantages of a sanitising device according to the invention are clear, which can be installed on view or in covered spaces, which is able to fit into a layout of a conventional means of public transport without the need for special systems and which can be modulated and applied on different vehicles. In particular, the sanitisation system just described has the following advantageous aspects in terms of
- Efficacy: the WO3 tungsten trioxide catalyst is more efficient in pathogen abatement than the titanium dioxide photocatalyst working on a wider spectrum;
- Safety: the system not only works with ultraviolet light that requires adequate protection and shielding of the source, but is activated with visible light and can therefore also work continuously with passengers on board;
- Cost effectiveness: light sources with visible light are relatively inexpensive compared to UV sources;
- Durability: the system does not lose its effectiveness over time because the active elements are not subject to consumption, given that the electrons of tungsten trioxide are reacquired in contact with the air;
- Modularity and scalability: the device is modular thanks to the use of a single standard lamellar module that can be combined in both longitudinal and transverse directions; the light source, when formed by a flexible strip, can also be scalable as required.

The present invention is not limited to the particular embodiments previously described and illustrated in the accompanying drawings, but numerous detail changes may be made thereto, within the reach of the person skilled in the art, without thereby departing from the scope of the invention itself, as defined in the appended claims.

## Claims

1. Device (1; 201) for sanitising by photocatalysis the air inside a vehicle, in particular a means of public transport, such as a bus or the like, comprising:
- a removable support structure (14) designed to be mounted at the ends of air delivery and intake ducts inside the vehicle;
- at least one lamellar module (100) formed by a perimeter frame (110) supporting a plurality of lamellae (101) placed parallel one to the other and inclined with respect to the plane of said perimeter frame (110), said lamellae (101) having one face turned towards the air inlet on which a photocatalytic nanomaterial is applied;
- at least one light source (16) at said at least one lamellar module (100) capable of illuminating the face of said lamellae (101) on which the photocatalytic nanomaterial is applied;
wherein said at least one lamellar module (100) and said at least one light source (16) are carried by the support structure (14),
**characterised in that** said photocatalytic nanomaterial is tungsten trioxide (WO₃), said support structure (14) supports several lamellar modules (100) joined one to the other in lamellar assemblies (200) and said perimeter frame (110) of each lamellar module (100) has the outer edge shaped, comprising recesses (111) and protrusions (112) in such a way that it can be coupled on any side to another lamellar module (100) in order to form said lamellar assemblies (200).

2. Device (1; 201) according to claim 1, wherein said at least one light source comprises several lighting units (16) each arranged at a respective lamella (101) of said at least one lamellar module (100).

3. Device (1; 201) according to claim 1 or 2, wherein said light source (16) is an LED light.

4. Device (1; 201) according to claim 3, wherein said LED light source (16) is applied to the support structure (14) in the form of a flexible LED strip.

5. Device (1; 201) according to any one of the previous claims, wherein said support structure (14) has a pair of opposing and symmetrical elements, each comprising a first face (13), said first faces (13) being designed to support on opposite sides the perimeter frame (110) of said at least one lamellar module (100), and each comprising one second face (12), inclined with respect to the first face (13), for fixing to the vehicle structure.

6. Device (1; 201) according to claim 5, wherein each element of said support structure (14) is made in two pieces and has one element provided with said first face (13) with L section and an element provided with said second face (12) with S section.

7. Device (1) according to any one of the preceding claims, in which said support structure (14) is coupled to a base (10) having openings (15) for the aspiration of air on which a perforated grille (11) is installed.

## Patentansprüche

1. Vorrichtung (1; 201) zum Reinigen der Luft innerhalb eines Fahrzeugs, insbesondere eines Mittels des öffentlichen Transports, wie beispielsweise eines Busses oder dergleichen, durch Photokatalyse, wobei die Vorrichtung Folgendes umfasst:
- eine entfernbare Tragstruktur (14), die dafür ausgelegt ist, an den Enden von Luftzufuhr- und -ansaugkanälen innerhalb des Fahrzeugs angebracht zu werden,
- wenigstens ein Lamellenmodul (100), das durch einen Einfassungsrahmen (110) gebildet wird, der eine Vielzahl von Lamellen (101) trägt, die parallel zueinander und geneigt in Bezug auf die Ebene des Einfassungsrahmens (110) angeordnet sind, wobei die Lamellen (101) eine Fläche aufweisen, die zu dem Lufteinlass hin gedreht ist, auf die ein photokatalytisches Nanomaterial aufgebracht ist,
- wenigstens eine Lichtquelle (16) an dem wenigstens einen Lamellenmodul (100), die dazu in der Lage ist, die Fläche der Lamellen (101), auf die das photokatalytische Nanomaterial aufgebracht ist, zu beleuchten, wobei das wenigstens eine Lamellenmodul (100) und die wenigstens eine Lichtquelle (16) durch die Tragstruktur (14) getragen werden,
**dadurch gekennzeichnet, dass** das photokatalytische Material Wolframtrioxid (WO₃) ist, die Tragstruktur (14) mehrere Lamellenmodule (100) trägt, die in Lamellenbaugruppen (200) miteinander verbunden sind, und der Einfassungsrahmen (110) jedes Lamellenmoduls (100) die Außenkante geformt hat, wobei sie Vertiefungen (111) und Vorsprünge (112) auf eine solche Weise umfasst, dass es auf einer beliebigen Seite mit einem anderen Lamellenmodul (100) gekoppelt werden kann, um die Lamellenbaugruppen (200) zu bilden.

2. Vorrichtung (1; 201) nach Anspruch 1, wobei die wenigstens eine Lichtquelle mehrere Beleuchtungseinheiten (16) umfasst, die jeweils an einer jeweiligen Lamelle (101) des wenigstens einen Lamellenmoduls (100) angeordnet sind.

3. Vorrichtung (1; 201) nach Anspruch 1 oder 2, wobei die Lichtquelle (16) eine LED-Leuchte ist.

4. Vorrichtung (1; 201) nach Anspruch 3, wobei die LED-Lichtquelle (16) in der Form eines flexiblen LED-Streifens auf die Tragstruktur (14) aufgebracht ist.

5. Vorrichtung (1; 201) nach einem der vorhergehenden Ansprüche, wobei die Tragstruktur (14) ein Paar von gegenüberliegenden und symmetrischen Elementen aufweist, die jeweils eine erste Fläche (13) umfassen, wobei die ersten Flächen (13) dafür ausgelegt sind, auf entgegengesetzten Seiten den Einfassungsrahmen (110) des wenigstens einen Lamellenmoduls (100) zu tragen, undjeweils eine zweite Fläche (12), die in Bezug auf die erste Fläche (13) geneigt ist, zum Befestigen an der Fahrzeugstruktur umfassen.

6. Vorrichtung (1; 201) nach Anspruch 5, wobei jedes Element der Tragstruktur (14) in zwei Teilen hergestellt ist und ein Element, das mit der ersten Fläche (13) versehen ist, mit einem L-Querschnitt und ein Element, das mit der zweiten Fläche (12) versehen ist, mit einem S-Querschnitt aufweist.

7. Vorrichtung (1) nach einem der vorhergehenden Ansprüche, wobei die Tragstruktur (14) an eine Basis (10) gekoppelt ist, die Öffnungen (15) für das Ansaugen von Luft aufweist, auf denen ein perforiertes Gitter (11) installiert ist.

## Revendications

1. Dispositif (1 ; 201) d'assainissement par photocatalyse de l'air à l'intérieur d'un véhicule, notamment un moyen de transport en commun, tel qu'un bus ou analogue, comprenant :
- une structure de support amovible (14) destinée à être montée aux extrémités des conduits de refoulement et d'admission d'air à l'intérieur du véhicule ;
- au moins un module lamellaire (100) formé par un cadre périmétrique (110) supportant une pluralité de lamelles (101) placées parallèlement les unes aux autres et inclinées par rapport au plan dudit cadre périmétrique (110), lesdites lamelles (101) ayant une face tournée vers l'entrée d'air sur laquelle est appliqué un nanomatériau photocatalytique ;
- au moins une source lumineuse (16) au niveau dudit au moins un module lamellaire (100) capable d'éclairer la face desdites lamelles (101) sur laquelle est appliqué le nanomatériau photocatalytique ; dans lequel ledit au moins un module lamellaire (100) et ladite au moins une source lumineuse (16) sont portés par la structure de support (14),
**caractérisé en ce que** ledit nanomatériau photocatalytique est du trioxyde de tungstène (WO₃), ladite structure de support (14) supporte plusieurs modules lamellaires (100) reliés les uns aux autres en assemblages lamellaires (200) et ledit cadre périmétrique (110) de chaque module lamellaire (100) a le bord extérieur formé, comprenant des évidements (111) et des protubérances (112) de telle manière qu'il puisse être couplé de n'importe quel côté à un autre module lamellaire (100) afin de former lesdits ensembles lamellaires (200).

2. Dispositif (1 ; 201) selon la revendication 1, dans lequel ladite au moins une source lumineuse comprend plusieurs unités d'éclairage (16) agencées chacune au niveau d'une lamelle respective (101) dudit au moins un module lamellaire (100).

3. Dispositif (1 ; 201) selon la revendication 1 ou 2, dans lequel ladite source lumineuse (16) est une lumière LED.

4. Dispositif (1 ; 201) selon la revendication 3, dans lequel ladite source lumineuse LED (16) est appliquée sur la structure de support (14) sous la forme d'une bande LED flexible.

5. Dispositif (1 ; 201) selon une quelconque des revendications précédentes, dans lequel ladite structure de support (14) comporte une paire d'éléments opposés et symétriques, comprenant chacun une première face (13), lesdites premières faces (13) étant conçues pour soutenir sur des faces opposées le cadre périmétrique (110) dudit au moins un module lamellaire (100), et comprenant chacune une deuxième face (12), inclinée par rapport à la première face (13), en vue de la fixation à la structure du véhicule.

6. Dispositif (1 ; 201) selon la revendication 5, dans lequel chaque élément de ladite structure de support (14) est réalisé en deux pièces et comporte un élément pourvu de ladite première face (13) avec une section en L et un élément pourvu de ladite deuxième face (12) avec une section en S.

7. Dispositif (1) selon une quelconque des revendications précédentes, dans lequel ladite structure de support (14) est couplée à une base (10) présentant des ouvertures (15) d'aspiration d'air sur lesquelles une grille perforée (11) est installée.
